# EUROPEAN PATENT APPLICATION

(11) **EP 4 321 622 A1**
(43) Date of publication of application: **14.02.2024**
(21) Application number: 22784997.3
(22) Date of filing: 07.04.2022
(51) Int. Cl.: C12N 15/77, C07K 14/34, C12P 13/10, C12R 1/15

(54) **L-ARGININE-PRODUCING CORYNEBACTERIUM SP. MICROORGANISM AND L-ARGININE PRODUCTION METHOD USING SAME**

(30) Priority: 07.04.2021 KR 20210045262
(71) Applicant: CJ Cheiljedang Corporation, Seoul 04560 (KR)
(72) Inventor: KIM, Hyo Kyung, Seoul 04560 (KR); CHANG, Jin Sook, Seoul 04560 (KR); CHOI, Sun Hyoung, Seoul 04560 (KR); OH, Haena, Seoul 04560 (KR); YOON, Byoung Hoon, Seoul 04560 (KR); KIM, Hyung Joon, Seoul 04560 (KR)
(74) Representative: Regimbeau
(86) International application number: PCT/KR2022/005056
(87) International publication number: WO 2022/216088

(57) **Abstract**

The present disclosure relates to an L-arginine-producing microorganism, in which a protein comprising an amino acid sequence of SEQ ID NO: 1 is weakened, and a method for producing L-arginine using the same.

## Description

### [Technical Field]

The present disclosure relates to an L-arginine-producing microorganism, in which a protein including an amino acid sequence of SEQ ID NO: 1 is weakened; and a method for producing L-arginine using the same.

### [Background Art]

L-Arginine has been widely used in medicines as a hepatic function-promoting agent, as a brain function-promoting agent, and as an ingredient of multiple amino acid supplements. Additionally, L-arginine has gained much interest in the food industry as a food additive for fish cakes and health beverages, and as a salt substitute for hypertension patients. Studies have been continuously conducted to use microorganisms for the production of industrially available high-concentration arginine, and examples thereof include a method of using a mutant strain induced from the microorganism belonging to the genus *Brevibacterium* or *Corynebacterium,* which is a glutamate-producing strain, or a method of using an amino acid-producing strain whose growth is improved through cell fusion. Meanwhile, lysE of the microorganism belonging to the genus *Corynebacterium* having an ability to export L-lysine has also been shown to export the same basic amino acid L-arginine (Bellmann A, et al., Microbiology, 147:1765-1774, 2001). Further, it has been disclosed a method for enhancing the production ability of L-arginine-producing strains through gene enhancement (U.S. Patent Application Publication No. US 2003-0113899 A1).

### [Disclosure]

### [Technical Problem]

The present inventors have developed a microorganism of the genus *Corynebacterium* producing L-arginine and a method for producing L-arginine using the same, thereby completing the present disclosure.

### [Technical Solution]

It is one object of the present disclosure to provide a recombinant microorganism of the genus *Corynebacterium* producing L-arginine, in which the activity of a protein including an amino acid sequence of SEQ ID NO: 1 is weakened.

It is another object of the present disclosure to provide a method for producing L-arginine, including: culturing a recombinant microorganism of the genus *Corynebacterium,* in which the activity of a protein including an amino acid sequence of SEQ ID NO: 1 is weakened, in a medium.

### [Advantageous Effects]

The microorganism of the genus *Corynebacterium,* in which the activity of a protein including an amino acid sequence of SEQ ID NO: 1 is weakened, can produce L-arginine in high yield, and thus can be effectively used for industrial production of L-arginine.

### [Detailed Description of Preferred Embodiments]

The present disclosure will be described in detail as follows. Meanwhile, each description and embodiment disclosed herein can be applied to other descriptions and embodiments, respectively. That is, all combinations of various elements disclosed herein fall within the scope of the present disclosure. Further, the scope of the present disclosure is not limited by the specific description described below. Additionally, a number of papers and patent documents have been cited throughout the present specification. The content of the cited papers and patent documents is incorporated herein by reference in their entirety and the level of technical field to which the present invention belongs and the contents of the present invention will be described more clearly.

One aspect of the present disclosure provides a recombinant microorganism of the genus *Corynebacterium,* in which the activity of a protein including an amino acid sequence of SEQ ID NO: 1 is weakened.

The protein of the present disclosure may have, include, consist or essentially consist of an amino acid represented by SEQ ID NO: 1.

In the present discourse, the protein including the amino acid sequence of SEQ ID NO: 1 may be an endogenous protein of the microorganism of the present disclosure, but is not limited thereto.

The amino acid sequence of SEQ ID NO: 1 can be obtained from NIH GenBank, a known database. In the present disclosure, the amino acid sequence of SEQ ID NO: 1 may include an amino acid sequence having a homology or identity of at least 70%, 75%, 80%, 85%, 90%, 95%, 96%, 97%, 98%, 99%, 99.5%, 99.7% or 99.9% with the amino acid sequence of SEQ ID NO: 1. Additionally, it is apparent that variants having an amino acid sequence, in which a part of the amino acid sequence is deleted, modified, substituted, conservatively substituted or added, may fall within the scope of the present disclosure, as long as the amino acid sequence has such homology or identity and shows an efficacy corresponding to that of the variant of the present disclosure.

For example, it may include sequence additions or deletions, naturally occurring mutations, silent mutations or conservative substitutions that do not alter the function of the variant of the present disclosure at the N-terminus, C-terminus and/or within the amino acid sequence.

As used herein, the term "conservative substitution" refers to substitution of an amino acid with another amino acid having similar structural and/or chemical properties. The variant may have, for example, one or more conservative substitutions while still retaining one or more biological activities. Such amino acid substitution may generally occur based on similarity of polarity, charge, solubility, hydrophobicity, hydrophilicity, and/or amphipathic nature of a residue. For example, among the amino acids having an electrically charged side chain (electrically charged amino acid), positively charged (basic) amino acids include arginine, lysine, and histidine; negatively charged (acidic) amino acids include glutamic acid and aspartic acid. Further, among the amino acids having an uncharged side chain (uncharged amino acid), nonpolar amino acids include glycine, alanine, valine, leucine, isoleucine, methionine, phenylalanine, tryptophan, and proline; polar or hydrophilic amino acids include serine, threonine, cysteine, tyrosine, asparagine, and glutamine; and among the nonpolar amino acids, aromatic amino acids include phenylalanine, tryptophan and tyrosine.

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of conserved polynucleotide or proteins may be determined by standard alignment algorithms and can be used with a default gap penalty established by the program being used. Substantially, homologous or identical sequences are generally expected to hybridize to all or part of the entire length of the sequences under moderate or high stringent conditions. It is apparent that hybridization with polynucleotides containing general codon or degenerate codons in hybridizing polynucleotides is also included.

Whether any two polynucleotide or protein sequences have a homology, similarity or identity may be, for example, determined by a known computer algorithm such as the "FASTA" program using default parameters (Pearson et al., (1988) Proc. Natl. Acad. Sci. USA 85:2444). Alternatively, it may be determined by the Needleman-Wunsch algorithm (Needleman and Wunsch, 1970, J. Mol. Biol. 48:443-453), which is performed using the Needleman program of the EMBOSS package (EMBOSS: The European Molecular Biology Open Software Suite, Rice et al., 2000, Trends Genet. 16:276-277) (version 5.0.0 or later) (GCG program package (Devereux, J., et al., Nucleic Acids Research 12:387 (1984)), BLASTP, BLASTN, FASTA (Atschul, S. F., et al., J MOLEC BIOL 215:403 (1990); Guide to Huge Computers, Martin J. Bishop, ed., Academic Press, San Diego, 1994, and CARILLO et al. (1988) SIAM J Applied Math 48:1073). For example, the homology, similarity or identity may be determined using BLAST or ClustalW of the National Center for Biotechnology Information (NCBI).

The homology, similarity, or identity of polynucleotides or proteins may be determined by comparing sequence information using, for example, the GAP computer program, such as Needleman et al. (1970), J Mol Biol. 48:443 as disclosed in Smith and Waterman, Adv. Appl. Math (1981) 2:482. In summary, the GAP program defines the homology, similarity or identity as the value obtained by dividing the number of similarly aligned symbols (*i.e*., nucleotides or amino acids) by the total number of the symbols in the shorter of the two sequences. Default parameters for the GAP program may include (1) a unary comparison matrix (containing a value of 1 for identities and 0 for non-identities) and the weighted comparison matrix of Gribskov et al. (1986), Nucl. Acids Res. 14:6745, as disclosed in Schwartz and Dayhoff, eds., Atlas of Protein Sequence and Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

In the present disclosure, the polynucleotide encoding the protein including the amino acid sequence of SEQ ID NO: 1 may be named NCgl2608 gene.

As used herein, the term "polynucleotide", which is a polymer of nucleotides composed of nucleotide monomers connected in a lengthy chain by a covalently bond, is a DNA or RNA strand having at least a certain length. More specifically, it may refer to a polynucleotide fragment encoding the variant.

The polynucleotide encoding the variant of the present disclosure may include a nucleotide sequence encoding the amino acid sequence represented by SEQ ID NO: 1. As an example of the present disclosure, the polynucleotide of the present disclosure may have or include a nucleotide sequence of SEQ ID NO: 2. In addition, the polynucleotide of the present disclosure may consist or consist essentially of a nucleotide sequence of SEQ ID NO: 2.

The polynucleotide of the present disclosure may undergo various modifications in the coding region within the scope that does not change the amino acid sequence of the variant, due to codon degeneracy or in consideration of the codons preferred in an organism in which the variant of the present disclosure is to be expressed. Specifically, the polynucleotide of the present disclosure may have or include a nucleotide sequence having a homology or identity of 70% or more, 75% or more, 80% or more, 85% or more, 90% or more, 95% or more, 96% or more, 97% or more, 98% or more and less than 100% with the sequence of SEQ ID NO: 2, or may consist or consist essentially of a nucleotide sequence having a homology or identity of 70% or more, 75% or more, 80% or more, 85% or more, 90% or more, 95% or more, 96% or more, 97% or more, 98% or more, and less than 100% with the sequence of SEQ ID NO: 2, but is not limited thereto.

Additionally, the polynucleotide of the present disclosure may include a probe that may be prepared from a known gene sequence, for example, any sequence which can hybridize with a sequence complementary to all or part of the polynucleotide sequence of the present disclosure under stringent without limitation. The "stringent conditions" refers to conditions under which specific hybridization between polynucleotides is allowed. Such conditions are specifically described in the literature (J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, the stringent conditions may include conditions under which polynucleotides having a high homology or identity of 70% or more, 75% or more, 80% or more, 85% or more, 90% or more, 95% or more, 96% or more, 97% or more, 98% or more, or 99% or more are hybridized with each other and polynucleotides having a homology or identity lower than the above homologies or identities are not hybridized with each other, or washing conditions of Southern hybridization, that is, washing once, specifically, twice or three times at a salt concentration and a temperature corresponding to 60°C, 1× SSC, 0.1% SDS, specifically 60°C, 0.1× SSC, 0.1% SDS, and more specifically 68°C, 0.1 × SSC, 0.1% SDS.

Hybridization requires that two nucleic acids contain complementary sequences, although mismatches between bases are possible depending on the stringency of the hybridization. The term "complementary" is used to describe the relationship between nucleotide bases that can hybridize with each other. For example, with respect to DNA, adenine is complementary to thymine, and cytosine is complementary to guanine. Therefore, the polynucleotide of the present disclosure may include isolated nucleotide fragments complementary to the entire sequence as well as nucleic acid sequences substantially similar thereto.

Specifically, polynucleotides having a homology or identity with the polynucleotide of the present disclosure may be detected using the hybridization conditions including a hybridization step at a Tₘ value of 55°C under the above-described conditions. Further, the Tₘ value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (*e.g*., Sambrook *et al*.).

As used herein, the term "microorganism (or strain)" includes all wild-type microorganisms, or naturally or artificially genetically modified microorganisms, and it may be a microorganism in which a particular mechanism is weakened or enhanced due to insertion of a foreign gene, or enhancement or inactivation of the activity of an endogenous gene, and may be a microorganism including genetic modification to produce a desired protein or product.

The microorganism of the present disclosure may be a microorganism in which the protein including the amino acid sequence of SEQ ID NO: 1 of the present disclosure or the polynucleotide encoding the protein is deleted; or a microorganism (*i.e*., a recombinant microorganism) which is genetically modified through a vector such that the protein including the amino acid sequence of SEQ ID NO: 1 of the present disclosure or the polynucleotide encoding the protein is deleted, but is not limited thereto.

The microorganism of the present disclosure may be a microorganism having the ability to produce L-arginine.

The microorganism of the present disclosure may be a microorganism having increased L-arginine producing ability compared to a parent strain.

The microorganism of the present disclosure may be a microorganism in which the ability to produce L-arginine has been imparted by weakening the protein including the amino acid sequence of SEQ ID NO: 1 of the present disclosure or deleting the polynucleotide encoding the protein into a parent strain (*i.e*., having the ability to naturally produce L-arginine or having no ability to produce L-arginine), but is not limited thereto.

In one example, the recombinant microorganism of the present disclosure is a microorganism which is transformed with a vector such that the protein including the amino acid sequence of SEQ ID NO: 1 of the present disclosure is weakened or the polynucleotide encoding the protein is deleted, and thus the protein including the amino acid sequence of SEQ ID NO: 1 of the present disclosure is weakened or the polynucleotide encoding the protein is deleted, and may be a microorganism having increased L-arginine producing ability as compared to a natural wild-type microorganism by weakening the protein including the amino acid sequence of SEQ ID NO: 1 of the present disclosure or deleting the polynucleotide encoding the protein in a natural wild-type microorganism or an L-arginine-producing microorganism, or a microorganism in which the protein including the amino acid sequence of SEQ ID NO: 1 of the present disclosure or the polynucleotide encoding the protein is increased as compared to a non-modified microorganism, but is not limited thereto.

In one example, the non-modified microorganism, which is a target strain to be compared to determine the increase in the L-arginine producing ability, may be a *Corynebacterium glutamicum* KCCM10741P (van der Rest et al., Appl Microbiol Biotechnol 52:541-545, 1999), or a strain in which the *argR* gene is deleted in the wild-type *Corynebacterium glutamicum* ATCC 13869 (*e.g*., *Corynebacterium glutamicum* CJ1R, but is not limited thereto.

In one example, the recombinant strain having increased producing ability may have an increased L-arginine producing ability by about 1% or more, specifically about 1% or more, about 2.5% or more, about 5% or more, about 6% or more, about 7% or more, about 8% or more, about 9% or more, about 10% or more, about 10.5% or more, about 11% or more, about 11.5% or more, about 12% or more, about 12.5% or more, about 13% or more, about 13.5% or more, or about 14% or more (the upper limit is not particularly limited, for example, about 200% or less, about 150% or less, about 100% or less, about 50% or less, about 40% or less, about 30% or less, or about 25% or less), as compared to that of the parent strain before modification or a non-modified microorganism, but is not limited thereto, as long as it has an increased + value compared to the production ability of the parent strain before modification or non-modified microorganism. In another example, the microorganism having increased production ability may have an increased L-arginine producing ability by about 1.1 times or more, about 1.12 times or more, about 1.13 times or more, or 1.14 times or more (the upper limit is not particularly limited, for example, about 10 times or less, about 5 times or less, about 3 times or less, or about 2 times or less), as compared to that of the parent strain before modification or non-modified microorganism, but is not limited thereto. As used herein, the term "about" refers to a range which includes all of ±0.5, ±0.4, ±0.3, ±0.2, ±0.1, *etc.* and includes all of the values that are equivalent or similar to those following the values, but the range is not limited thereto.

As used herein, the term "non-modified microorganism" does not exclude a strain containing a mutation that may occur naturally in a microorganism, and may refer to a wild-type strain or a natural-type strain itself, or a strain before the trait is altered due to genetic modification caused by natural or artificial factors. For example, the non-modified microorganism may refer to a microorganism before the protein including the amino acid sequence of SEQ ID NO: 1 of the present disclosure described in the present specification is weakened or the polynucleotide encoding the protein is deleted. The "non-modified microorganism" may be used interchangeably with "strain before modification", "microorganism before modification", "non-mutant strain", "non-modified strain", "non-mutant microorganism", or "reference microorganism".

In another embodiment, the microorganism of the genus *Corynebacterium* of the present disclosure may be *Corynebacterium glutamicum, Corynebacterium crudilactis, Corynebacterium deserti, Corynebacterium efficiens, Corynebacterium callunae, Corynebacterium stationis, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens.*

Specifically, the recombinant microorganism of the present disclosure may be a microorganism in which all or part of the polynucleotide encoding the protein consisting of the amino acid sequence of SEQ ID NO: 1 is deleted.

Additionally, the microorganism of the present disclosure may be a microorganism in which the activity of arginine repressor (ArgR) is additionally weakened. Specifically, the microorganism of the present disclosure may be a microorganism in which all or part of the *argR* gene is additionally deleted.

The ArgR may include a polypeptide represented by the amino acid sequence of SEQ ID NO: 13. In addition, the *argR* gene may include a polynucleotide represented by the nucleotide sequence of SEQ ID NO: 14, but is not limited thereto.

In addition, the microorganism of the present disclosure may be a microorganism in which the activity of carbamoyl phosphate synthase small subunit (CarA) and/or carbamoyl phosphate synthase large subunit (CarB) are further enhanced, or which expresses normal ornithine carbamoyltransferase F (ArgF), or includes a combination thereof.

In one example, the microorganism of the present disclosure a *Corynebacterium glutamicum* microorganism in which carA and/or carB protein activity is further enhanced in NCgl2608 gene-deleted strain of the *Corynebacterium glutamicum* KCCM10741P (van der Rest et al., Appl Microbiol Biotechnol 52:541-545, 1999) or NCgl2608 gene-deleted strain of the *Corynebacterium glutamicum* CJ1R.

In addition, the microorganism of the present disclosure may be a strain in which the NCgl2608 gene is deleted in *Corynebacterium glutamicum* KCCM10741P, or a microorganism that additionally expresses normal argF in *Corynebacterium glutamicum* CJ1R, and the microorganism expressing the normal argF additionally be a *Corynebacterium glutamicum* microorganism with additionally enhanced carA and/or carB protein activity.

The argF protein may contain a polypeptide represented by the amino acid sequence of SEQ ID NO: 15, and the *argF* gene may contain a polynucleotide represented by the nucleotide sequence of SEQ ID NO: 16, but are not limited thereto. Further, the carA protein may contain a polypeptide represented by the amino acid sequence of SEQ ID NO: 17, the carB protein may contain a polypeptide represented by the amino acid sequence of SEQ ID NO: 19, the *carA* gene may contain a polynucleotide represented by the nucleotide sequence of SEQ ID NO: 18, and the *carB* gene may contain a polynucleotide represented by the nucleotide sequence of SEQ ID NO: 20.

As used herein, the term "weakening" of a protein is a comprehensive concept including both reduced or no activity compared to its endogenous activity. The weakening may be used interchangeably with terms such as inactivation, deficiency, down-regulation, decrease, reduce, attenuation, *etc.*

The weakening may also include a case where the protein activity itself is decreased or removed compared to the activity of the protein originally possessed by a microorganism due to a mutation of the polynucleotide encoding the protein; a case where the overall level of intracellular protein activity and/or concentration (expression level) is decreased compared to a natural strain due to the inhibition of expression of the gene of the polynucleotide encoding the protein, or the inhibition of translation into the protein, *etc.;* a case where the polynucleotide is not expressed at all; and/or a case where no protein activity is observed even when the polynucleotide is expressed. As used herein, the term "endogenous activity" refers to the activity of a particular protein originally possessed by a parent strain before transformation, a wild-type or a non-modified microorganism, when a trait is altered through genetic modification caused by natural or artificial factors, and may be used interchangeably with "activity before modification". The expression "the protein activity is 'inactivated, deficient, decreased, down-regulated, reduced or attenuated' compared to its endogenous activity" means that the protein activity is decreased compared to the activity of a particular protein originally possessed by a parent strain before transformation or a non-modified microorganism.

The weakening of the protein activity can be performed by any method known in the art, but the method is not limited thereto, and can be achieved by applying various methods well known in the art (*e.g*., Nakashima N et al., Bacterial cellular engineering by genome editing and gene silencing. Int J Mol Sci. 2014;15(2):2773-2793; Sambrook et al. Molecular Cloning 2012; *etc.*).

Specifically, the weakening of the protein activity of the present disclosure may be achieved by:
1) deleting a part or all of the gene encoding the protein;
2) modifying the expression regulatory region (expression regulatory sequence) such that the expression of the gene encoding the protein is decreased;
3) modifying the amino acid sequence constituting the protein such that the protein activity is removed or weakened (*e.g*., deletion/substitution/addition of one or more amino acids on the amino acid sequence);
4) modifying the gene sequence encoding the protein such that the protein activity is removed or weakened (*e.g*., deletion/substitution/addition of one or more of nucleotides on the nucleotide sequence of the protein gene to encode a protein that has been modified to remove or weaken the activity of the protein);
5) modifying the nucleotide sequence encoding the initiation codon or 5'-UTR of the gene transcript encoding the protein;
6) introducing an antisense oligonucleotide (*e.g*., antisense RNA), which binds complementary to the gene transcript encoding the protein;
7) adding a sequence complementary to the Shine-Dalgarno (SD) sequence on the front end of the SD sequence of the gene encoding the protein to form a secondary structure, thereby inhibiting the ribosomal attachment;
8) a reverse transcription engineering (RTE), which adds a promoter, which is to be reversely transcribed, on the 3' terminus of the open reading frame (ORF) of the gene sequence encoding the protein; or
9) a combination of two or more selected from the methods 1) to 8) above, but is not particularly limited thereto.

For example,
The 1) method of deleting a part or all of the gene encoding the protein may be achieved by deleting all of the polynucleotide encoding the endogenous target protein within the chromosome, or by replacing the polynucleotide with a polynucleotide or a marker gene having partially deleted nucleotides.

The 2) method of modifying the expression regulatory region (expression regulatory sequence) may be achieved by inducing a modification on the expression regulatory region (expression regulatory sequence) through deletion, insertion, non-conservative substitution or conservative substitution, or a combination thereof; or by replacing the sequence with a sequence having a weaker activity. The expression regulatory region may include a promoter, an operator sequence, a sequence encoding a ribosome-binding site, and a sequence for regulating transcription and translation, but is not limited thereto.

The 3) method of modifying the nucleotide sequence encoding the initiation codon or 5'-UTR of the gene transcript encoding the protein may be achieved, for example, by substituting the nucleotide sequence with a nucleotide sequence encoding another initiation codon having a lower polypeptide expression rate than the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the protein or the polynucleotide sequence encoding the protein, or a combination thereof to weaken the activity of the protein, or by replacing the sequence with an amino acid sequence or a polynucleotide sequence modified to have a weaker activity, or an amino acid sequence or a polynucleotide sequence modified to have no activity, but are not limited thereto. For example, the expression of a gene may be inhibited or weakened by introducing a mutation into the polynucleotide sequence to form a stop codon, but is not limited thereto.

The 6) method of introducing an antisense oligonucleotide (*e.g*., antisense RNA), which binds complementary to the gene transcript encoding the protein can be found in the literature (Weintraub, H. et al., Antisense-RNA as a molecular tool for genetic analysis, Reviews - Trends in Genetics, Vol. 1(1) 1986).

The 7) method of adding a sequence complementary to the Shine-Dalgarno (SD) sequence on the front end of the SD sequence of the gene encoding the protein to form a secondary structure, thereby inhibiting the ribosome attachment may be achieved by inhibiting mRNA translation or reducing the speed thereof.

The 8) reverse transcription engineering (RTE), which adds a promoter, which is to be reversely transcribed, on the 3' terminus of the open reading frame (ORF) of the gene sequence encoding the protein may be achieved by forming an antisense nucleotide complementary to the gene transcript encoding the protein to weaken the activity.

As used herein, the term "enhancement" of a protein activity means that the activity of a protein is increased compared to its endogenous activity. The enhancement may be used interchangeably with terms such as activation, up-regulation, overexpression, increase, *etc.* In particular, the terms activation, enhancement, up-regulation, overexpression and increase may include both cases in which an activity not originally possessed is exhibited, or the activity is enhanced compared to the endogenous activity or the activity before modification. The "endogenous activity" refers to the activity of a particular protein originally possessed by a parent strain before transformation or a non-modified microorganism, when a trait is altered through genetic modification caused by natural or artificial factors, and may be used interchangeably with "activity before modification". The "enhancement", "up-regulation", "overexpression", or "increase" in the activity of a protein compared to its endogenous activity means that the activity and/or concentration (expression level) of the protein is enhanced compared to that of a particular protein originally possessed by a parent strain before transformation or a non-modified microorganism.

The enhancement may be achieved by introducing a foreign protein, or by enhancing the activity and/or concentration (expression level) of the endogenous protein. The enhancement of the activity of the protein can be confirmed by the increase in the level of activity of the protein, expression level, or the amount of product excreted from the protein.

The enhancement of the activity of the protein can be applied by various methods well known in the art, and is not limited as long as it can enhance the activity of the target protein compared to that of the microorganism before modification. Specifically, genetic engineering and/or protein engineering well known to those skilled in the art, which is a common method of molecular biology, may be used, but the method is not limited thereto (*e.g*., Sitnicka et al. Functional Analysis of Genes. Advances in Cell Biology. 2010, Vol. 2. 1-16; Sambrook et al. Molecular Cloning 2012; *etc.*).

Specifically, the enhancement of the protein of the present disclosure may be achieved by:
1) increasing the intracellular copy number of a polynucleotide encoding the protein;
2) replacing the expression regulatory region of a gene encoding the protein on the chromosome with a sequence having a strong activity;
3) modifying the nucleotide sequence encoding the initiation codon or 5'-UTR of the gene transcript encoding the protein;
4) modifying the amino acid sequence of the protein such that the activity of the polypeptide is enhanced;
5) modifying the polynucleotide sequence encoding the protein such that the activity of the protein is enhanced (*e.g*., modifying the polynucleotide sequence of the protein gene to encode a protein that has been modified to enhance the activity of the protein);
6) introducing a foreign protein having the activity of the protein or a foreign polynucleotide encoding the same;
7) codon-optimization of the polynucleotide encoding the protein;
8) analyzing the tertiary structure of the protein and thereby selecting and modifying the exposed site, or chemically modifying the same; or
9) a combination of two or more selected from above 1 to 8), but is not particularly limited thereto.

More specifically,
The 1) method of increasing the intracellular copy number of a polynucleotide encoding the protein may be achieved by introducing a vector, which is operably linked to the polynucleotide encoding the protein and is able to replicate and function regardless of a host cell, into the host cell. Alternatively, the method may be achieved by introducing one copy or two copies of polynucleotides encoding the protein into the chromosome of a host cell. The introduction into the chromosome may be performed by introducing a vector, which is able to insert the polynucleotide into the chromosome of a host cell, into the host cell, but is not limited thereto. The vector is as described above.

The 2) method of replacing the expression regulatory region (or expression regulatory sequence) of a gene encoding the protein on the chromosome with a sequence having a strong activity may be achieved by inducing a modification on the sequence through deletion, insertion, non-conservative or conservative substitution, or a combination thereof to further enhance the activity of the expression regulatory region, or by replacing the sequence with a sequence having a stronger activity. The expression regulatory region may include, but is not particularly limited to, a promoter, an operator sequence, a sequence encoding a ribosome-binding site, and a sequence regulating the termination of transcription and translation. In one example, the method may include replacing the original promoter with a strong promoter, but is not limited thereto.

Examples of the strong promoter may include CJ1 to CJ7 promoters (US 7662943 B2), lac promoter, trp promoter, trc promoter, tac promoter, lambda phage PR promoter, PL promoter, tet promoter, gapA promoter, SPL7 promoter, SPL13(sm3) promoter (US 10584338 B2), O2 promoter (US 10273491 B2), tkt promoter, yccA promoter, *etc.,* but are not limited thereto.

The 3) method of modifying the nucleotide sequence encoding the initiation codon or 5'-UTR of the gene transcript encoding the protein may be achieved, for example, by substituting the nucleotide sequence with a nucleotide sequence encoding another initiation codon having a higher expression rate of the polypeptide compared to the endogenous initiation codon, but is not limited thereto.

The 4) and 5) methods of modifying the amino acid sequence or the polynucleotide sequence may be achieved by inducing a modification on the sequence through deletion, insertion, non-conservative or conservative substitution of the amino acid sequence of the protein or the polynucleotide sequence encoding the protein, or a combination thereof to enhance the activity of the protein, or by replacing the sequence with an amino acid sequence or polynucleotide sequence modified to have a stronger activity, or an amino acid sequence or polynucleotide sequence modified to enhance the activity, but are not limited thereto. The replacement may specifically be performed by inserting the polynucleotide into the chromosome by homologous recombination, but is not limited thereto. The vector used herein may further include a selection marker to confirm the insertion into the chromosome. The selection marker is the same as described above.

The 6) method of introducing a foreign polynucleotide having the activity of the protein may be achieved by introducing into a host cell a foreign polynucleotide encoding a protein that exhibits the same or similar activity to the protein. The foreign polynucleotide may be used without limitation regardless of its origin or sequence as long as it exhibits the same or similar activity to the protein. The introduction may be performed by those of ordinary skill in the art by appropriately selecting a transformation method known in the art, and the expression of the introduced polynucleotide in the host cell enables to produce the protein, thereby increasing its activity.

The 7) method of codon-optimization of the polynucleotide encoding the protein may be achieved by codon-optimization of an endogenous polynucleotide to increase the transcription or translation within a host cell, or by optimizing the codons thereof such that the optimized transcription and translation of the foreign polynucleotide can be achieved within the host cell.

In addition, the 8) method of analyzing the tertiary structure of the protein and thereby selecting and modifying the exposed site, or chemically modifying the same may be achieved, for example, by comparing the sequence information of the protein to be analyzed with a database, in which the sequence information of known proteins is stored, to determine template protein candidates according to the degree of sequence similarity, and thus confirming the structure based on the information, thereby selecting and transforming or modifying the exposed site to be modified or chemically modified.

Such enhancement of the protein activity may mean that the activity or concentration (expression level) of the protein is increased relative to the activity or concentration of the protein expressed in a wild-type or a microorganism before modification, or that the amount of product produced from the protein is increased, but is not limited thereto.

As used herein, the term "vector" may include a DNA construct containing the nucleotide sequence of a polynucleotide encoding the target polypeptide operably linked to a suitable expression regulatory region (expression regulatory sequence) so as to be able to express the target polypeptide in a suitable host cell. The expression regulatory sequence may include a promoter capable of initiating transcription, any operator sequence for regulating the transcription, a sequence encoding a suitable mRNA ribosome-binding site, and a sequence for regulating termination of transcription and translation. Once transformed into a suitable host cell, the vector may replicate or function independently from the host genome, or may integrate into genome thereof.

The vector used in the present disclosure is not particularly limited, and any vector known in the art may be used. Examples of the vector typically used may include natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, *etc.* may be used; and as a plasmid vector, those based on pDC, pBR, pUC, pBluescriptll, pGEM, pTZ, pCL and pET, *etc.* may be used. Specifically, pDZ, pDC, pDCM2, pACYC177, pACYC184, pCL, pECCG117, pUC19, pBR322, pMW118, pCC1BAC vector, *etc.* may be used.

In one example, a polynucleotide encoding a target polypeptide may be inserted into the chromosome through a vector for intracellular chromosomal insertion. The insertion of the polynucleotide into the chromosome may be performed by any method known in the art, for example, by homologous recombination, but is not limited thereto. The vector may further include a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cell toxic agents, or expression of surface proteins, may be used. Only cells expressing the selection marker are able to survive or to show different phenotypes under the environment treated with the selective agent, and thus the transformed cells may be selected.

As used herein, the term "transformation" refers to the introduction of a vector containing a polynucleotide encoding a target polypeptide into a host cell or a microorganism so that the polypeptide encoded by the polynucleotide can be expressed in the host cell. As long as the transformed polynucleotide can be expressed in the host cell, it does not matter whether the transformed polynucleotide is integrated into the chromosome of the host cell and located therein or located extrachromosomally, and both cases can be included. Further, the polynucleotide may include DNA and/or RNA encoding the target polypeptide. The polynucleotide may be introduced in any form, as long as it can be introduced into the host cell and expressed therein. For example, the polynucleotide may be introduced into the host cell in the form of an expression cassette, which is a gene construct including all elements required for its autonomous expression. The expression cassette may commonly include a promoter operably linked to the polynucleotide, a transcription terminator, a ribosome-binding site, or a translation terminator. The expression cassette may be in the form of a self-replicable expression vector. Additionally, the polynucleotide may be introduced into the host cell as it is and operably linked to sequences required for expression in the host cell, but is not limited thereto.

Further, as used herein, the term "operably linked" means that the polynucleotide sequence is functionally linked to a promoter sequence that initiates and mediates transcription of the polynucleotide encoding the target variant of the present disclosure.

The modification of a part or all of the polynucleotide in the microorganism of the present disclosure may be achieved by (a) homologous recombination using a vector for chromosomal insertion in the microorganism or genome editing using engineered nuclease (*e.g*., CRISPR-Cas9) and/or (b) may be induced by light, such as ultraviolet rays and radiation, *etc.,* and/or chemical treatments, but is not limited thereto. The method of modifying a part or all of the gene may include a method using DNA recombination technology. For example, a part or all of the gene may be deleted by injecting a nucleotide sequence or a vector containing a nucleotide sequence homologous to the target gene into a microorganism to induce homologous recombination. The injected nucleotide sequence or the vector may include a dominant selection marker, but is not limited thereto.

Another aspect of the present disclosure provides a method for producing L-arginine, including: culturing a recombinant microorganism of the genus *Corynebacterium,* in which the activity of a protein including an amino acid sequence of SEQ ID NO: 1 is weakened, in a medium.

The protein including the amino acid sequence of SEQ ID NO: 1, weakening, and microorganism, *etc.* are the same as described in the other aspects above.

The microorganism of the genus *Corynebacterium* may be *Corynebacterium glutamicum,* but is not limited thereto.

The microorganism may be a microorganism in which the activity of arginine repressor (ArgR) is further weakened or the *argR* gene is further deleted, but is not limited thereto, and this is as described in the other aspects above.

As used herein, the term "cultivation" means that the microorganism of the genus *Corynebacterium* of the present disclosure is grown under appropriately controlled environmental conditions. The cultivation process of the present disclosure may be performed in a suitable culture medium and culture conditions known in the art. Such a cultivation process may be easily adjusted for use by those skilled in the art according to the strain to be selected. Specifically, the cultivation may be a batch culture, a continuous culture, and/or a fed-batch culture, but is not limited thereto.

As used herein, the term "medium" refers to a mixture of materials which contains nutrient materials required for the cultivation of the microorganism of the genus *Corynebacterium* of the present disclosure as a main ingredient, and it supplies nutrient materials and growth factors, along with water that is essential for survival and growth. Specifically, the medium and other culture conditions used for culturing the microorganism of the genus *Corynebacterium* of the present disclosure may be any medium used for conventional cultivation of microorganisms without any particular limitation. However, the microorganism of the genus *Corynebacterium* of the present disclosure may be cultured under aerobic conditions in a conventional medium containing an appropriate carbon source, nitrogen source, phosphorus source, inorganic compound, amino acid, and/or vitamin, while adjusting temperature, pH, *etc.*

Specifically, the culture medium for the microorganism of the genus *Corynebacterium* can be found in the literature ("Manual of Methods for General Bacteriology" by the American Society for Bacteriology (Washington D.C., USA, 1981)).

In the present disclosure, the carbon source may include carbohydrates, such as glucose, saccharose, lactose, fructose, sucrose, maltose, *etc.;* sugar alcohols, such as mannitol, sorbitol, *etc.;* organic acids, such as pyruvic acid, lactic acid, citric acid, *etc.;* amino acids, such as glutamic acid, methionine, lysine, *etc.* Additionally, the carbon source may include natural organic nutrients such as starch hydrolysate, molasses, blackstrap molasses, rice bran, cassava, sugar cane molasses, corn steep liquor, *etc.* Specifically, carbohydrates such as glucose and sterilized pretreated molasses (*i.e.*, molasses converted to reducing sugar) may be used, and in addition, various other carbon sources in an appropriate amount may be used without limitation. These carbon sources may be used alone or in a combination of two or more kinds, but are not limited thereto.

The nitrogen source may include inorganic nitrogen sources, such as ammonia, ammonium sulfate, ammonium chloride, ammonium acetate, ammonium phosphate, ammonium carbonate, ammonium nitrate, *etc*.; amino acids, such as glutamic acid, methionine, glutamine, *etc.;* and organic nitrogen sources, such as peptone, NZ-amine, meat extract, yeast extract, malt extract, corn steep liquor, casein hydrolysate, fish or decomposition product thereof, defatted soybean cake or decomposition product thereof, *etc.* These nitrogen sources may be used alone or in a combination of two or more kinds, but are not limited thereto.

The phosphorus source may include monopotassium phosphate, dipotassium phosphate, or corresponding sodium-containing salts, *etc.* Examples of the inorganic compound may include sodium chloride, calcium chloride, iron chloride, magnesium sulfate, iron sulfate, manganese sulfate, calcium carbonate, *etc.* Additionally, amino acids, vitamins, and/or appropriate precursors, *etc.* may be included. These constituting ingredients or precursors may be added to a medium in a batch or continuous manner, but these phosphorus sources are not limited thereto.

Further, the pH of a medium may be adjusted during the cultivation of the microorganism of the genus *Corynebacterium* of the present disclosure by adding a compound such as ammonium hydroxide, potassium hydroxide, ammonia, phosphoric acid, sulfuric acid, *etc.* to the medium in an appropriate manner. Additionally, during the cultivation, an antifoaming agent such as fatty acid polyglycol ester may be added to prevent foam generation. In addition, oxygen or oxygencontaining gas may be injected into the medium in order to maintain an aerobic state of the medium; or nitrogen, hydrogen, or carbon dioxide gas may be injected without the injection of gas in order to maintain an anaerobic or microaerobic state of the medium, but the gas is not limited thereto.

The medium temperature in the cultivation of the present disclosure may be in a range from 20°C to 45°C, and specifically, from 25°C to 40°C, and the cultivation may be carried out for about 10 to 160 hours, but is not limited thereto.

The L-arginine produced by the cultivation of the present disclosure may be released into the medium or remain in the cells.

The method for producing L-arginine of the present disclosure may further include a step of preparing the microorganism of the genus *Corynebacterium* of the present disclosure, a step of preparing a medium for culturing the microorganism, or a combination thereof (regardless of the order, in any order), for example, prior to the culturing step.

The method for producing L-arginine of the present disclosure may further include a step of recovering L-arginine from the culture medium (medium on which the culture was grown) or the cultured microorganism of the genus *Corynebacterium.* The recovering step may be further included after the culturing step.

In the recovering step, desired L-arginine may be collected using the method of culturing a microorganism of the present disclosure, for example, using a suitable method known in the art according to a batch culture, continuous culture, or fed-batch culture method. For example, methods such as centrifugation, filtration, treatment with a protein crystallizing precipitant (salting-out method), extraction, ultrasonic disruption, ultrafiltration, dialysis, various kinds of chromatography such as molecular sieve chromatography (gel filtration), adsorption chromatography, ion exchange chromatography, affinity chromatography, *etc.,* HPLC, or a combination thereof may be used, and the desired L-arginine can be recovered from the medium or microorganisms using suitable methods known in the art.

Further, the method for producing L-arginine of the present disclosure may further include a purification process, which may be performed using an appropriate method known in the art. In one example, when the method for producing L-arginine of the present disclosure includes both a recovering step and a purification step, the recovering step and the purification step may be performed continuously or intermittently regardless of the order, or simultaneously or may be integrated into one step, but the method is not limited thereto.

Still another aspect of the present disclosure provides a composition for producing L-arginine, including: a microorganism of the genus *Corynebacterium,* in which the activity of the protein including the amino acid sequence of SEQ ID NO: 1 of the present disclosure is weakened; a medium for culturing the same; or a combination thereof.

The composition of the present disclosure may further include any suitable excipient commonly used in the composition for producing amino acids, and such excipient may be, for example, a preservative, a wetting agent, a dispersing agent, a suspending agent, a buffering agent, a stabilizing agent, or an isotonic agent, *etc.*, but is not limited thereto.

In the composition of the present disclosure, the protein including the amino acid sequence of SEQ ID NO: 1, weakening, microorganism, cultivation, and medium, *etc.* are the same as described in the other aspects above.

Yet another aspect of the present disclosure provides a method for producing a microorganism of the genus *Corynebacterium,* including weakening a protein including an amino acid sequence of SEQ ID NO: 1.

Even another aspect of the present disclosure provides the use of L-arginine production of a microorganism of the genus *Corynebacterium,* in which the activity of a protein including an amino acid sequence of SEQ ID NO: 1 is weakened.

In the method for producing the microorganism of the genus *Corynebacterium* and the use of L-arginine production of the microorganism of the genus *Corynebacterium,* the protein including the amino acid sequence of SEQ ID NO: 1, weakening, and microorganism, *etc.* are the same as described in the other aspects above.

### [Mode for Carrying Out the Invention]

Hereinafter, the present disclosure will be described in detail by way of Examples. However, these Examples are merely preferred Examples given for illustrative purposes, and thus, the scope of the present disclosure is not intended to be limited to or by these Examples. Meanwhile, technical features which are not described herein can be sufficiently understood and easily carried out by those skilled in the art in the technical field of the present disclosure or in a similar technical field.

### Example 1: Construction of Random Mutation Library using Transposon and Screening Thereof

In order to obtain a strain having increased L-arginine producing ability, the plasmids obtained using EZ-Tn5^{™} <R6Kyori/KAN-2>Tnp Transposome^{™} Kit (Epicentre) were transformed based on the *Corynebacterium glutamicum* KCCM10741P (Korean Patent No. 0791659) as the parent strain by electroporation *(*Appl. Microbiol. Biotechnol. (1999) 52:541-545) and spread on a complex medium plate containing kanamycin (25 mg/L) to thereby obtain about 20,000 colonies.

### <Complex Medium Plate (pH 7.0)>

Glucose 10 g, Peptone 10 g, Beef extract 5 g, Yeast extract 5 g, Brain Heart Infusion 18.5 g, NaCl 2.5 g, Urea 2 g, Sorbitol 91 g, Aga 20 g (per liter of distilled water)

The top 10 mutant strains with improved L-arginine producing ability compared to the *Corynebacterium glutamicum* KCCM10741P, the parent strain, were selected using the ninhydrin method (Moore, S., Stein, W. H., Photometric ninhydrin method for use in the chromatography of amino acids. J. Biol. Chem. 1948, 176, 367-388).

### Example 2: Analysis of L-Arginine Producing Ability of Selected Random Mutant Strains

In order to finally select strains with reproducibly increased L-arginine producing ability among the 10 mutant strains selected in Example 1, flask culture was performed using the following medium. After completion of the culture, the L-arginine concentration in the culture medium was analyzed using HPLC, and the L-arginine production concentration of each mutant strain is shown in Table 1 below.

### <Production Medium (pH 7.0)>

Glucose 6%, Ammonium sulfate 3%, Monopotassium phosphate 0.1%, MgSO₄·7H₂O 0.2%, CSL (corn steep liquor) 1.5%, NaCl 1%, Yeast extract 0.5%, Biotin 100 mg/L, CaCO₃ 3%, (per liter of distilled water).

**[Table 1]**

| L-Arginine Production Concentration of 10 Selected Randon Mutant Strains | | | | | |
|---|---|---|---|---|---|
| | Strain | L-Arginine (g/L) | | | |
| | | Batch 1 | Batch 2 | Batch 3 | Average |
| Control | KCCM10741P | 3.1 | 3.2 | 3 | 3.1 |
| 1 | KCCM10741P/mt-1 | 2.9 | 3 | 2.8 | 2.9 |
| 2 | KCCM10741P/mt-2 | 3 | 2.9 | 2.7 | 2.9 |
| 3 | KCCM10741P/mt-3 | 3.1 | 3 | 3.1 | 3.1 |
| 4 | KCCM10741P/mt-4 | 2.8 | 2.9 | 3.2 | 3.0 |
| 5 | KCCM10741P/mt-5 | 3.5 | 3.5 | 3.4 | 3.5 |
| 6 | KCCM10741P/mt-6 | 2.9 | 3.1 | 3.2 | 3.1 |
| 7 | KCCM10741P/mt-7 | 3 | 3.2 | 2.8 | 3.0 |
| 8 | KCCM10741P/mt-8 | 2.9 | 2.9 | 3 | 2.9 |
| 9 | KCCM10741P/mt-9 | 3.1 | 3.1 | 2.8 | 3.0 |
| 10 | KCCM10741P/mt-10 | 3 | 2.9 | 2.9 | 2.9 |

Among the 10 mutant strains selected, KCCM10741P/mt-5 was finally selected as a strain with significantly improved L-arginine producing ability.

### Example 3: Identification of Causes of Increase in L-Arginine Producing Ability of Finally Selected Strains

The gene inactivated by random insertion of a transposon in the KCCM10741P/mt-5 of Example 2 was identified.

Specifically, the genomic DNA of KCCM10741P/mt-5 was extracted, cleaved and ligated to transform into *E*. *coli* DH5α, and plated on LB solid medium containing kanamycin (25 mg/L). After selecting 20 types of transformed colonies, a plasmid containing part of an unknown gene was obtained. As a result of analyzing the nucleotide sequence using primer 1 (SEQ ID NO: 3) and primer 2 (SEQ ID NO: 4) of the EZ-Tn5^{™} <R6Kyori/KAN-2> Tnp Transposome^{™} Kit, it was found that the gene including the nucleotide sequence of SEQ ID NO: 2 was inactivated based on the nucleotide sequence registered in the NIH GenBank of the National Institutes of Health.

**[Table 2]**

| Primers Used | Nucleotide Sequences |
|---|---|
| Primer 1(SEQ ID NO: 3) | ACCTACAACAAAGCTCTCATCAACC |
| Primer 2(SEQ ID NO: 4) | CTACCCTGTGGAACACCTACATCT |

As a result of a BLAST search of SEQ ID NO: 2 of the wild-type *Corynebacterium glutamicum* ATCC 13869, it was confirmed that it was the NCgl2608 gene.

### Example 4: Construction of Recombinant Vector for NCgl2608 Gene Deletion

In order to reconfirm the effect of inactivation of the gene including the nucleotide sequence of SEQ ID NO: 2 on L-arginine production, a recombinant vector was constructed for deletion of the NCgl2608 gene on the chromosome of a strain of the genus *Corynebacterium.*

First, primers 3 to 6 were synthesized to construct fragments for deletion of the above genes, and these are shown in Table 3.

**[Table 3]**

| Primers 3 to 6 for Production of Fragments for Gene Deletion | |
|---|---|
| Primers Used | Nucleotide Sequences |
| Primer 3 (SEQ ID NO: 5) | tcggtacccggggatccTCGGTGGCGGTTTCGG C |
| Primer 4 (SEQ ID NO: 6) | TTGCAGAAAGGGATACCAGGGCTAGCTC ATAGAC |
| Primer 5 (SEQ ID NO: 7) | GGTATCCCTTTCTGCAACGATGTGCTGTG TGACG |
| Primer 6 (SEQ ID NO: 8) | tgcaggtcgactctagaAGGACCTGGAAAAGGG C |

In order to delete the ORF region based on SEQ ID NO: 2, primer 3 (SEQ ID NO: 5), primer 4 (SEQ ID NO: 6), primer 5 (SEQ ID NO: No. 7), and primer 6 (SEQ ID NO: 8) (Table 2) were synthesized to have BamHI restriction enzyme site at the 5' end and Xbal restriction enzyme site at the 3' end, and PCR was performed based on the chromosomal DNA of the wild-type *Corynebacterium glutamicum* ATCC 13869 as a template (Sambrook et al., Molecular Cloning, a Laboratory Manual (1989), Cold Spring Harbor Laboratories). Based on this, a DNA fragment was obtained in which the top 600 bp and the bottom 600 bp of the portion encoding the protein encoded by the nucleotide sequence of NCgl2608 were connected. In particular, the PCR was performed under PCR conditions of 30 cycles of denaturation at 95°C for 30 seconds; annealing at 50°C for 30 seconds; and polymerization at 72°C for 1 minutes, and then polymerization at 72°C for 7 minutes. The pDZ vector (Korean Patent No. 10-0924065), which cannot replicate in *Corynebacterium glutamicum,* and the fragment amplified by PCR were treated with restriction enzymes BamHI and Xbal and ligated using a DNA ligation enzyme, and transformed into *E*. *coli* DH5α and plated on LB solid medium containing kanamycin (25 mg/L).

After selecting colonies transformed with the plasmid into which the target gene was inserted through PCR, a plasmid was obtained using a plasmid extraction method, and this plasmid was named pDZ-ΔNCgl2608.

### Example 5: Construction of Strain with Deleted NCgl2608 Gene Derived from Corynebacterium glutamicum KCCM10741P and Evaluation of L-Arginine Producing Ability

pDZ-ANCgl2608 was transformed into *Corynebacterium glutamicum* KCCM10741P, an L-arginine producing strain, by homologous recombination on the chromosome (van der Rest et al., Appl Microbiol Biotechnol 52:541-545, 1999).

Thereafter, secondary recombination was performed on a solid plate medium containing 4% sucrose. A strain with deleted gene of SEQ ID NO: 2 on the chromosome was identified through PCR using primers 3 and 6 based on the *Corynebacterium glutamicum* transformants for which secondary recombination was completed. The recombinant strain was named *Corynebacterium glutamicum* KCCM10741P-ΔNCgl2608.

In order to analyze the L-arginine producing ability of the *Corynebacterium glutamicum* KCCM10741P-ΔNCgl2608 strain constructed above, the strain was cultured with *Corynebacterium glutamicum* KCCM10741P, the parent strain, in the following manner.

The *Corynebacterium glutamicum* KCCM10741P, the parent strain, and the *Corynebacterium glutamicum* KCCM10741P-ΔNCgl2608 strain constructed in Example 6 were inoculated into a 250 mL corner-baffle flask containing 25 mL of the production medium below, and then cultured with shaking at 200 rpm at 30°C for 20 hours. Then, 1 mL of seed culture was inoculated into a 250 mL corner-baffle flask containing 24 mL of production medium and cultured under shaking at 200 rpm at 30°C for 72 hours. The compositions of the seed medium and production medium are as follows.

### <Seed Medium (pH 7.0)>

Glucose 20 g, Pepetone 10 g, Yeast extract 5 g, Urea 1.5 g, KH₂PO₄ 4 g, K₂HPO₄ 8 g, MgSO₄·7H₂O 0.5 g, Biotine 100 µg, Thiamine-HCl 1000 µg, Calcium pantothenate 2000 µg, Nicotinamide 2000 µg (per liter of distilled water)

### <Production Medium (pH 7.0)>

Glucose 6%, Ammonium sulfate 3%, Monopotassium phosphate 0.1%, MgSO₄·7H₂O 0.2%, CSL (corn steep liquor) 1.5%, NaCl 1%, Yeast extract 0.5%, Biotin 100 mg/L

After completion of the culture, the production amount of L-arginine was measured by HPLC (Waters 2478), and the concentration of L-arginine analyzed is shown in Table 4 below.

**[Table 4]**

| Analysis of L-Arginine Producing Ability of KCCM10741P-derived KCCM10741P-ΔNCgl2608 | | | | | |
|---|---|---|---|---|---|
| | Strains | L-Arginine (g/L) | | | |
| | | Batch 1 | Batch 2 | Batch 3 | Average |
| Control | KCCM10741P | 3 | 2.9 | 3.1 | 3.0 |
| Experiment al group | KCCM10741P-ΔNCgl2608 | 3.5 | 3.7 | 3.6 | 3.6 |

As shown in the results above, when the gene including the nucleotide sequence of SEQ ID NO: 2 was deleted from the *Corynebacterium glutamicum* KCCM10741P, an L-arginine-producing strain, it was confirmed that the L-arginine producing ability was increased by an average of 20.0% compared to the parent strain.

Accordingly, it was confirmed that L-arginine producing ability can be improved by deleting the gene including the nucleotide sequence of SEQ ID NO: 2 in the microorganism of the genus *Corynebacterium.*

### Example 6: Construction of Strain with Deleted NCgl2608 Gene Derived from Corynebacterium glutamicum CJ1R and Evaluation of L-Arginine Producing Ability

In order to confirm whether the same effect could be obtained in other strains of *Corynebacterium glutamicum* that produce L-arginine, an attempt was made to introduce one type of mutation (Δ*argR*) into the wild strain (ATCC 13869).

The recombinant vector for deletion of the *argR* gene was constructed in the same manner as Example 4, and the primers used to construct the vector are shown in Table 5

**[Table 5]**

| Primers 7 to 10 for Production of Fragments for Deletion of *argR* gene | |
|---|---|
| Primers Used | Nucleotide Sequences |
| Primer 7 (SEQ ID NO: 9) | tcggtacccggggatccAGCAGGCCTTAAGGGTAAG |
| Primer 8 (SEQ ID NO: 10) | AGGGGCGCTGTCTTACCTCGGCTGGTGGGCCAGC |
| Primer 9 (SEQ ID NO: 11) | GGTAAGACAGCGCCCCTAGTTCAAGGCTTGTTAATC |
| Primer 10 (SEQ ID NO: 12) | tgcaggtcgactctagaACCGTTGAACTGCTTGCCAG |

After selecting the plasmid into which the target gene was inserted through PCR, this plasmid was named pDZ-ΔargR. The wild-type *Corynebacterium glutamicum* strain was transformed using pDZ-ΔargR in the same manner as in Example 6, and the strain with *argR* deletion on the chromosome was identified through PCR using primers 7 and 10 based on the transformants. The recombinant strain was named CJ1R, and a strain with deleted NCgl2608 gene was constructed based on the *Corynebacterium glutamicum* CJ1R, which had arginine producing ability, in the same manner as in Example 6, and then was named CJ1R-ΔNCgl2608.

The CJ1R-ΔNCgl2608 with deleted NCgl2608 gene was cultured in the same manner as in Example 5 above, and after completion of the culture, the production of L-arginine was measured by HPLC (Waters 2478), and the concentration of L-arginine analyzed is shown in Table 6 below.

**[Table 6]**

| L-Arginine Producing Ability of CJ1R-Derived CJ1R-ΔNCgl2608 | | | | | |
|---|---|---|---|---|---|
| | Strains | L-Arginine (g/L) | | | |
| | | Batch 1 | Batch 2 | Batch 3 | Average |
| Control | CJ1R | 1.3 | 1.3 | 1.4 | 1.33 |
| Experimental group | CJ1R-ΔNCgl2608 | 1.6 | 1.5 | 1.6 | 1.56 |

As shown in the results above, it was confirmed that when the NCgl2608 gene was deleted from the *Corynebacterium glutamicum* CJ1R, an L-arginine producing strain, the L-arginine producing ability was increased by an average of 17.3%.

Accordingly, as in the results of Example 5, it was confirmed that L-arginine producing ability can be improved by deleting the NCgl2608 gene in the microorganism of the genus *Corynebacterium.*

The CJ1R-ΔNCgl2608 strain was named CA06-7204 and deposited at the Korean Culture Center of Microorganisms (KCCM), a Depositary Authority under the Budapest Treaty, on February 23, 2021, with Accession No. KCCM12961P.

From the foregoing, those of ordinary skill in the art to which the present disclosure belongs will recognize that the present disclosure may be embodied in other specific forms without departing from its spirit or essential characteristics. The described embodiments are to be considered in all respects only as illustrative and not restrictive. The scope of the present disclosure is therefore indicated by the appended claims rather than by the foregoing description. All changes which come within the meaning and range of equivalency of the claims are to be embraced within the scope of the present disclosure.

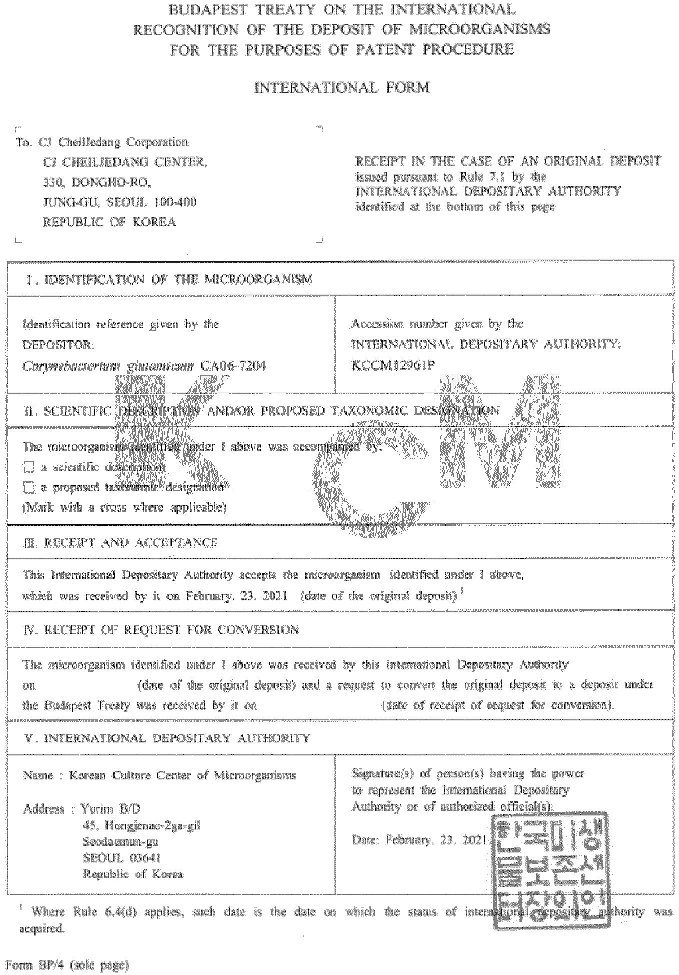
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA06-7204 | Accession number given by the
INTERNATIONAL DEPOSITARY AUTHORITY:
KCCM12961P

II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)

III. RECEIPT AND ACCEPTANCE
This International Depositary Authority accepts the microorganism identified under I above,
which was received by it on February. 23. 2021 (date of the original deposit)¹

IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: February. 23. 2021.


¹ Where Rule 6.4(d) applies, such date is the date on which the status of international depositary authority was
acquired.

Form BP/4 (sole page)

## Claims

1. A recombinant microorganism of the genus *Corynebacterium* producing L-arginine, in which the activity of a protein consisting of an amino acid sequence of SEQ ID NO: 1 is weakened.

2. The microorganism of claim 1, wherein the microorganism has increased L-arginine producing ability compared to a parent strain.

3. The microorganism of claim 1, wherein the microorganism is *Corynebacterium glutamicum.*

4. The microorganism of claim 1, wherein the activity of an arginine repressor is further weakened.

5. The microorganism of claim 1, wherein a polynucleotide encoding the protein consisting of the amino acid sequence of SEQ ID NO: 1 is deleted.

6. A method for producing L-arginine, comprising: culturing a recombinant microorganism of the genus *Corynebacterium,* in which the activity of a protein comprising an amino acid sequence of SEQ ID NO: 1 is weakened, in a medium.

7. The method of claim 6, wherein the microorganism is *Corynebacterium glutamicum.*

8. The method of claim 6, wherein the activity of an arginine repressor is further weakened in the microorganism of the genus *Corynebacterium.*

9. The method of claim 6, wherein a nucleic acid sequence encoding the protein consisting of the amino acid sequence of SEQ ID NO: 1 is deleted in the microorganism.
